(19) Europäisches Patentamt
European Patent Office
Office européen des brevets

(11) Veröffentlichungsnummer: **0 277 310**
**A1**

(12) **EUROPÄISCHE PATENTANMELDUNG**

(21) Anmeldenummer: **87117854.7**

(51) Int. Cl.4: **C07H 15/252**, A61K 31/70

(22) Anmeldetag: **03.12.87**

Patentansprüche für folgende Vertragsstaaten: ES + GR.

(30) Priorität: **08.12.86 DE 3641835**

(43) Veröffentlichungstag der Anmeldung: **10.08.88 Patentblatt 88/32**

(84) Benannte Vertragsstaaten: **AT BE CH DE ES FR GB GR IT LI LU NL SE**

(71) Anmelder: **BEHRINGWERKE AKTIENGESELLSCHAFT**
**Postfach 1140**
**D-3550 Marburg/Lahn(DE)**

(72) Erfinder: **Kolar, Cenek, Dr.**
**Deutschhausstrasse 20**
**D-3550 Marburg(DE)**
Erfinder: **Paal, Michael, Dr.**
**Eppendorfer Landstrasse 6B**
**D-2000 Hamburg 20(DE)**
Erfinder: **Hermentin, Peter, Dr.**
**Barfüssertor 30**
**D-3550 Marburg(DE)**
Erfinder: **Kraemer, Hans Peter, Dr.**
**Birkenweg 16**
**D-3550 Marburg(DE)**

(74) Vertreter: **Meyer-Dulheuer, Karl-Hermann, Dr. et al**
**HOECHST Aktiengesellschaft Zentrale**
**Patentabteilung Postfach 80 03 20**
**D-6230 Frankfurt/Main 80(DE)**

(54) **Zytostatisch wirksame Anthracyclinderivate.**

(57) Die Erfindung betrifft neue zytostatisch wirksame Anthracyclin-Derivate der allgemeinen Formel I

**EP 0 277 310 A1**

Xerox Copy Centre

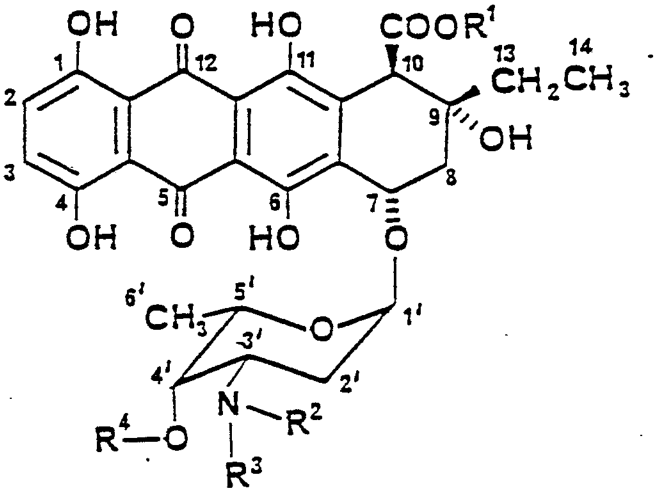

I

in der die Reste
$R^1$ eine $CH_3(CH_2)_n$ Gruppe mit n = 0 bis 3,
$R^2$ ein Wasserstoffatom oder eine Methylgruppe,
$R^3$ ein Wasserstoffatom, eine Methylgruppe oder eine Acylschutzgruppe,
$R^4$ ein Wasserstoffatom oder eine Acylschutzgruppe
bedeuten, und die gegebenenfalls als Salz einer anorganischen oder organischen Säure vorliegen, Verfahren zu ihrer Herstellung sowie ihre Verwendung in Arzneimitteln.

## Zytostatisch wirksame Anthracyclinderivate

Die Erfindung betrifft neue, zytostatisch wirksame Anthracyclinderivate, Verfahren zu ihrer Herstellung sowie ihre Verwendung in Arzneimitteln.

Die Substanzklasse der Anthracycline ist schon seit langem bekannt. Seit der Strukturermittlung der Rhodomycine, des Adriamycins bzw. des Daunomycins und dem Bekanntwerden der zytostatischen Wirksamkeit gewisser Vertreter dieser Anthracyclinklasse wurde eine Vielzahl von Anthracyclinen auf biologischem Wege aus Vertretern der Actinomyceten-Gattung Streptomyces gewonnen und in ihrer Wirkung erforscht.

Es ist bekannt, daß Anthracycline, in denen ein Daunosaminyl-oder Rhodosaminylbaustein vorkommt, Antitumoralwirkung aufweisen und daß beispielsweise Adriamycin und Aclacino mycin als Wirkstoffe in auf dem Markt befindlichen Produkten enthalten sind.

Aus einer Veröffentlichung von Essery und Doyle (Can. J. Chem. 58, 1869 (1980)) ist die Verknüfpung zwischen einem funktionalisierten Daunosamin und einem ε-Rhodomycinon bekannt. Das resultierende α-Glycosid weist indessen im Vergleich zu Adriamycin nur eine schwache zytostatische Wirksamkeit auf.

Überraschenderweise hat sich bei den von der Anmelderin durchgeführten umfangreichen Untersuchungen gezeigt, daß ε-Isorhodomycinon mit 1,4-Di-O-acetyl-L-rhodosamin glycosidiert werden kann, und daß das dabei gebildete 7-O-α-L-Rhodosaminyl-ε-isorhodomycinon bzw. sein 4'-O-Acetylderivat zytostatische Wirksamkeit besitzen,die mit der von Adriamycin vergleichbar ist.

Der vorliegenden Erfindung lag somit die Aufgabe zugrunde, ausgehend von dem auf biologischem Wege erhältlichen ε-Isorhodomycinon bzw. dessen in 10-Stellung entsprechend substituierten Derivaten neue Anthracyclin-Derivate zu schaffen, die sich durch zytostatische Wirksamkeit auszeichnen.

Die neuen erfindungsgemäßen Anthracyclin-Derivate entsprechen dabei der allgemeinen Formel I

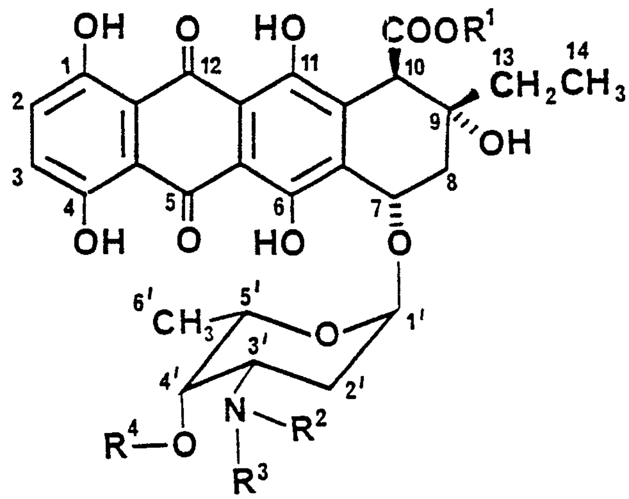

I

in der die Reste

$R^1$ eine $CH_3\,(CH_2)_n$ Gruppe mit n = 0 bis 3 ,

$R^2$ ein Wasserstoffatom oder eine Methylgruppe,

$R^3$ ein Wasserstoffatom, eine Methylgruppe oder eine in der Kohlehydratchemie übliche Acylschutzgruppe wie eine Acetyl-, Trifluoracetyl-, Benzoyl-oder para-Nitrobenzoylgruppe,

$R^4$ ein Wasserstoffatom oder eine Acylschutzgruppe bedeuten, und die gegebenenfalls als Salz einer anorganischen oder organischen Säure vorliegen, die selbstverständlich gesundheitlich verträglich ist.

Besonders bevorzugt werden im Rahmen der vorliegenden Erfindung ε-Isorhodomycinon-Glycosylkonjugate der vorgenannten allgemeinen Formel (I), in der die Reste

$R^1$ eine Methylgruppe,

$R^2$ ein Wasserstoffatom oder eine Methylgruppe,

$R^3$ ein Wasserstoffatom oder eine Methylgruppe, eine Trifluoracetyl-oder Acetylgruppe und

$R^4$ ein Wasserstoffatom, eine Acetyl-, Benzoyl-oder para-Nitrobenzoylgruppe bedeuten und die gegebenenfalls wieder als Salz einer organischen oder anorganischen Säure vorliegen können.

Das erfindungsgemäße Verfahren zur Herstellung der vorstehend beschriebenen neuen Anthracyclin-Derivate geht aus von dem auf biologischem Wege erhältlichen Anthracyclinon oder seinem in der 10-Stellung entsprechend veresterten Derivat der allgemeinen Formel II

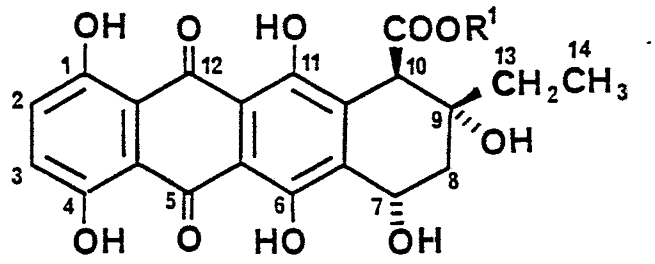

II

in der $R^1$ eine $CH_3(CH_2)_n$-Gruppe mit n gleich 0 bis 3 ist. Dieses Anthracyclinon-Derivat wird (a) mit einem funktionalisierten Daunosamin-Derivat der allgemeinen Formel III

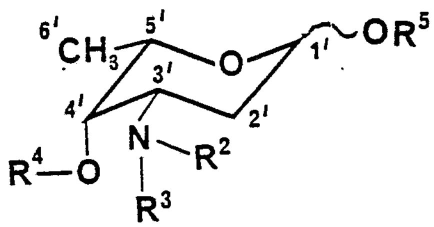

III

worin $R^2$ ein Wasserstoffatom oder eine Methylgruppe, $R^3$ eine Methylgruppe oder eine Acylschutzgruppe, $R^4$ eine Schutzgruppe, $R^5$ eine Acylschutzgruppe bedeuten, in Gegenwart eines organischen Lösungsmittels, eines Katalysators, gegebenenfalls eines Säurefängers und eines Trocken mittels bei einer Reaktionstemperatur von -70°C bis +30°C zu einer Verbindung der allgemeinen Formel (I) umgesetzt, wobei gegebenenfalls (b) bei einem Reaktionsprodukt der Stufe (a) in an sich bekannter Weise die Schutzgruppen am Kohlenhydratsegment mittels einer anorganischen oder organischen Base in einem Lösungsmittel partiell oder vollständig entblockiert und gegebenenfalls wieder selektiv an der 4'-Hydroxy-und/oder 3'-Amino-Gruppe oder 3'-Methylaminogruppe eine neue Acylschutzgruppe eingeführt wird, und gegebenenfalls (c) bei einem in Stufe (a) oder Stufe (b) erhaltenen 3'-N,N-Dimethylaminoreaktionsprodukt in an sich bekannter Weise auf photolytischem Wege in Gegenwart eines Lösungsmittels bei einer Reaktionstemperatur von +10°C bis +100°C eine am Stickstoff gebundene Methylgruppe abgespalten und gegebenenfalls das gebildete 3'-N-Methylamino-Derivat zu einem N-Acyl-Derivat oder einem N-Acyl-, O-Acyl-Derivat umgesetzt wird und gegebenenfalls (d) bei einem in Stufe (b) erhaltenen Daumosamin-Reaktionsprodukt in an sich bekannter Weise eine N-Methylierung zum 3-N-Methyldaunosamin-Reaktionsprodukt oder eine N,N-Dimethylierung zum Rhodosamin-Reaktionsprodukt durchgeführt wird und gegebenenfalls (e) ein Produkt der Stufe (a), (b), (c) oder (d) in an sich bekannter Weise in das Salz einer anorganischen Säure oder organischen Säure überführt wird.

Das im erfindungsgemäßen Verfahren eingesetzte Daunosamin-Derivat kann als Schutzgruppe beispielsweise als Rest $R^3$ eine in der Kohlehydratschemie übliche Acylschutzgruppe wie Acetyl-oder Trifluoracetylgruppe, als Rest $R^4$ eine in der Kohlehydratschemie übliche blockierende Acetyl-, Benzoyl-oder para-Nitrobenzoylgruppe und als Rest $R^5$ eine Acetyl-oder para-Nitrobenzoylgruppe tragen.

Als organisches Lösungsmittel, in dem die Reaktion durchgeführt wird, können beispielsweise Chloroform, Dichlormethan, Toluol, Äther, Dimethylformamid, Aceton, Aceto nitril oder deren Mischungen Anwendung finden, und als Katalysator kann beispielsweise Trifluormethansulfonsäuretrimethylsilylester benutzt werden. Als Säurefänger wird zweckmäßigerweise ein Molekularsieb 4 Å und als Trockenmittel beispielsweise Calciumsulfat verwendet.

Die Abspaltung der Schutzgruppen in der Stufe (b) kann mittels einer Base, wie Alkali-oder Erdalkalihydroxyden, Natriumcarbonat und Triäthylamin in einem Lösungsmittel wie Wasser, Methanol, Äthanol, THF oder deren Mischungen erfolgen.

Die in Stufe (c) durchgeführte photolytische Abspaltung einer Methylgruppe kann durch Einwirkung einer Lichtquelle, wie beispielsweise dem Sonnen-oder Tageslicht, oder eines starken Lichtstrahlers bewirkt werden, wobei als Lösungsmittel beispielsweise Chloroform, Methanol, Wasser oder deren Mischungen in

Betracht kommen.

Für die in Stufe (d) vorgesehene Methylierung der 3'-Aminogruppe können an sich beliebige Methylierungsmittel Anwendung finden. Als Beispiel sei für die Bildung der N,N-Dimethylverbindung die Methylierung mit 37%igem wäßrigen Formaldehyd und Natriumcyanoborhydrid angeführt.

Für die Salzbildung gemäß Verfahrensstufe (e) können an sich beliebige anorganische oder organische Säuren Verwendung finden, sofern sie im Hinblick auf die spätere Verwendung in Arzneimitteln gesundheitlich verträglich sind. Bevorzugte Anwendung finden Glutaminsäure oder Glucuronsäure.

Gemäß einer bevorzugten Ausführungsform des erfindungsgemäßen Verfahrens wird $\epsilon$-Isorhodomycinon mit 1,4-Di-O-acetyl-$\alpha$-L-rhodosamin in Gegenwart eines organischen Lösungsmittels, eines Katalysators, gegebenenfalls eines Säurefängers und eines Trockenmittels umgesetzt, gegebe nenfalls der photolytischen Abspaltung einer Methylgruppe, der Deacylierung und gegebenenfalls der erneuten Acylierung und Salzbildung unterworfen.

Gemäß einer anderen vorteilhaften Ausführungsform des erfindunsgemäßen Verfahrens wird $\epsilon$-Isorhodomycinon mit 1,4-di-O-Acetyl-3-N-acetyl-$\alpha$-L-daunosamin in Gegenwart eines organischen Lösungsmittels, eines Katalysators, gegebenenfalls eines Säurefängers und eines Trockenmittels umgesetzt, gegebenenfalls der Desacylierung und der N-Methylierung sowie gegebenenfalls der erneuten Acylierung und/oder Salzbildung unterworfen.

Die nach dem erfindungsgemäßen Verfahren erhaltenen neuen Anthracyclin-Derivate zeichnen sich wie bereits erwähnt durch zytostatische Aktivität aus und sei können daher zusammen mit den üblichen pharmazeutischen Konfektionierungs-und/oder Verdünnungsmittel zu Arzneimitteln verarbeitet werden, die in der Krebstherapie Anwendung finden. die Dosierungs-und Anwendungsweise entspricht dabei im wesentlichen derjenigen für die bekannten Substanzen Adriamycin, Daunomycin, Aclacinomycin, 4'-epi-Adriamycin, 4'-Methoxyadriamycin oder 4'-Desoxyadriamycin.

Die solchermaßen hergestellten Arzneimittel können zusätzlich noch andere Wirkstoffe enthalten, sofern diese zusammen mit den erfindungsgemäßen Verbindungen keine unerwünschten Nebenwirkungen zeigen.

Die zytostatische Wirksamkeit der erfindungsgemäßen Verbindungen wurde anhand von L1210 Leukämiezellen der Maus getestet. Hierzu wurde die Koloniebildung von L1210 Leukämiezellen in Soft Agar herangezogen. Diese Methode dient zum Nachweis des Einflusses der Testsubstanzen auf das Wachstumsverhalten der Zellen über mehrere Generationen. Bei einer Zellzykluszeit von 10 bis 12 Stunden werden dabei in der Testzeit von 7 Tagen etwa 14 aufeinanderfolgende Generationen beobachtet. Die erfindungsgemäßen zytostatisch wirksamen Substanzen bewirken in diesem Test eine Reduktion der zu beobachtenden Koloniezahl gegenüber einer unbehandelten Kontrollprobe.

Einzelheiten des Testverfahrens ergeben sich aus der nachfolgenden Verfahrensweise zur Ermittelung der Koloniebildung. Für die Ermittlung der akuten Toxizität der erfindungsgemäßen Anthracyclin-Derivate wurden entsprechende Tests an NMRI-Mäusen durchgeführt. Die Einzelheiten des Testverfahrens ergeben sich aus der nachfolgenden Verfahrensweise für die Ermittlung der akuten Toxizität.

Verfahrensweise für die Koloniebildung von L1210 Leukämiezellen in Soft Agar.

500 Leukämiezellen pro Platte werden mit unterschiedlichen Konzentrationen der Testsubstanz 1 Stunde bei 37°C inkubiert. Anschließend wurden die Zellen zweimal mit McCoy5A-Medium gewaschen und schließlich in Petrischalen nach Zusatz von 0,3 % Agar ausgegossen. Kontrollen wurden lediglich mit frischem Medium inkubiert. Anstelle der einstündigen Inkubation wurden in machen Fällen unterschiedliche Konzentrationen und Testsubstanzen der oberen Agarschicht zugemischt, um so eine kontinuierliche Exposition der Zellen über die gesamte Inkubationszeit zu erreichen. Nach Erstarren des Agars wurden die Platten im Brutschrank 7 Tage bei 37°C inkubiert (5 Vol-% $CO_2$, 95 % relative Luftfeuchtigkeit). Anschließend wurde die Zahl der entstandenen Kolonien mit einem Durchmesser von mehr als 60 $\mu$ gezählt. Die Ergebnisse wurden angegeben als Koloniezahl in behandelten Agarplatten in Prozent der unbehandelten Kontrolle. Aus der so erhaltenen Dosiswirkungskurve wurde die $IC_{50}$ als Maß für die Wirksamkeit der Substanz ermittelt. Die Ergebnisse für die hier beschriebenen Verbindungen im Vergleich zu Adriamycin sind in der Tabelle 1 zusammengefaßt.

Verfahrenweise für die Ermittlung der akuten Toxizität

Zur Ermittlung der akuten Toxizität wurden NMRI-Mäusen am Tag 0 unterschiedliche Dosen der Testsubstanz, gelöst in 0,5 ml physiologischer Kochsalzlösung, intravenös injiziert. Kontrollgruppen erhielten lediglich 0,5 ml physiologischer Kochsalzlösung. Pro Konzentration der Testsubstanz wurden 5 Mäuse verwendet. Am Tag 14 wurde die Zahl der überlebenden Mäuse ermittelt und daraus nach der Litchfield Wilcoxon Methode die LD50 ermittelt. Die Toxizität der hier beschriebenen Verbindungen im Vergleich zu Adriamycin ist in Tabelle 1 zusammengefaßt.

Tabelle 1

| Verbindung | Zytotoxizität (Dauerinkubation) $IC_{50}$ (ug/ml) | akute Toxizität $LD_{50}$ (mg/kg) |
|---|---|---|
| 7-O-(4'-O-Acetyl-α-L-rhodosaminyl)-ε-isorhodomycinon | 0,028 | 89,0 |
| 7-O-(α-L-Rhodosaminyl)-ε-isorhodomycinon | 0,035 | 50-100 |
| 7-O-(3'-N-Trifluoroacetyl-α-L-daunosaminyl)-ε-isorhodomycinon | 0,4 | - |
| 7-O-(4'-O-Acetyl-3'-N-methyl-α-L-daunosaminyl)-ε-isorhodomycinon | 0,1 | - |
| 7-O-(3'-N-Methyl-α-L-daunosaminyl)-ε-isorhodomycinon | 0,028 | - |
| Adriamycin | 0,02 | 26,5 |

Zur Erläuterung des erfindungsgemäßen Herstellungsverfahrens werden nachfolgend Beispiele 1 bis 11 aufgeführt, in denen bevorzugte erfindungsgemäße Verbindungen nach dem beanspruchten Verfahren hergestellt wurden.

Die Struktur der hergestellten Verbindungen wurde mittels $^1$H-und $^{13}$C-NMR sowie MS-oder IR-Spektroskopie ermittelt. Der Verlauf der Reaktionen sowie die resultierenden Verbindungen wurden dünnschichtchromatographisch oder mit der HPLC-Technik untersucht.

Beispiel 1:

7-O-(4'-O-Acetyl-α-L-rhodosaminyl)-ε-isorhodomycinon (Verbindung 1)

1 g (2,25 mmol) ε-Isorhodomycinon wurden in 200 ml wasserfreiem Dichlormethan gelöst. Zu dieser Lösung gab man bei Raumtemperatur 0,65 g (2,53 mmol) 1,4-Di-O-Acetyl-L-rhodosamin und 5 g Molekularsieb 4 Å in 20 ml wasserfreiem Dichlormethan und anschließend rührte man 0,5 h. Danach fügte man 0,6 ml Trifluormethansulfonsäuretrimethylsilylester hinzu. Nach 0,5 h wurde der Ansatz mit gesättigter Natriumhydrogencarbonatlösung versetzt. Die organische Phase wurde mit Wasser gewaschen, über Natriumsulfat getrocknet und im Vakuum (Wasserstrahlpumpe) eingeengt. Es erfolgte eine säulenchromatographische Reinigung an 80 g Kieselgel (Elutionsmittel: Toluol/Äthanol 10:1). Man erhielt 0,8 g (55% Ausbeute bezogen auf Aglycon) der Titelverbindung 1.
Schmelzpunkt: 223-224°C

Elementaranalyse Berechnet (%): C 59,71 H 5,79 N 2,17
Gefunden (%): C 59,97 H 5,81 N 2,03

Spektroskopische Daten $^1$H-NMR (400 MHz, $CDCl_3$, $\delta$): 5,68 (m, H-1'), 5,27 (dd, H-4'), 5,22 (dd, H-7), 4,49 (H-3'), 4,24 (S, H-10), 4,20 (H-5'), 3,70 (s, $OCH_3$), 2,19 (s, $N(CH_3)_2$), 2,18 (s, Ac).

Beispiel 2:

7-O-($\alpha$-L-Rhodosaminyl)-$\epsilon$ -isorhodomycinon (Verbindung 2)

0,5 g (0,77 mmol) Verbindung 1 wurden in 100 ml wasserfreiem Methanol gelöst und mit 0,1 ml 30 %iger Natriummethylatlösung versetzt. Nach 2 h Rühren bei Raumtemperatur wurde mit Ionenaustauscher Dowex WX8 neutralisiert. An schließend wurde abfiltriert und im Vakuum eingeengt. Es wurden 0,35 g (90%) der Titelverbindung 2 erhalten.

Elementaranalyse Berechnet (%) : C 59,89 H 5,86 N 2,32
Gefunden (%) : C 59,97 H 5,91 N 2,13

Spektroskopische Daten $^1$H-NMR (400 MHz, $CDCl_3$, $\delta$): 5,52 (dd, H-1'), 5,23 (H-7), 4,50 (H-9), 4,27 (H-10), 4,07 (H-5'), 3,73 ($OCH_3$), 2,25 ($N(CH_3)_2$).

Beispiel 3:

7-O-(4'-O-para Nitrobenzoyl-3-N-trifluoroacetyl-$\alpha$-L-daunosaminyl)-$\epsilon$-isorhodomycinon (Verbindung 3)

1,0 g (2,25 mmol) $\epsilon$-Isorhodomycinon wurden in 200 ml wasserfreiem Dichlormethan/Äther 1:1 gelöst, mit 5 g Molekularsieb 4 Å versetzt und 30 min bei Raumtemperatur gerührt. In die auf -20°C abgekühlte Suspension wurden 3,25 g (6 mmol) 1,4-Di-O-para-Nitrobenzoyl-3-N-trifluoroacetyl-L-daunosamin, gelöst in 50 ml Dichlormethan, zugegeben. Anschließend fügte man tropfenweise 1 ml Trifluoromethansulfonsäuretrimethylsilylester hinzu. Nach 30 Minuten wurde der Reaktionsansatz mit 2,2 ml Trimethylamin versetzt, und anschließend noch 10 Minuten gerührt. Das Reaktionsgemisch wurde abfiltriert und der Filterrückstand mit Dichlormethan nachgewaschen. Die organischen Lösungen wurden mit Wasser gewaschen und über wasserfreiem Natriumsulfat getrocknet. Der durch Abdampfen des Lösungsmittels erhaltene Rückstand wurde über Kieselgel chromatographiert. Es wurden 1,52 g (Ausbeute 82% bezogen auf Aglycon) Verbindung 3 erhalten.

Elementaranalyse Berechnet (%) : C 54,28 H 4,06 N 3,42 F 6,69
Gefunden (%) : C 54,02 H 4,06 N 3,28 F 6,80

Beispiel 4:

7-O-(4'-O-Acetyl-3'-N-trifluoroacetyl-α-L-daunosaminyl)-ε-isorhodomycinon (Verbindung 4)

Ausgehend von ε-Isorhodomycinon und 1,4-Di-O-Acetyl-3-N-trifluoroacetyl-L-daunosamin wurde die Verbindung 4, wie in der Vorschrift zur Herstellung der Verbindung 3 beschrieben, hergestellt, jedoch als Reaktionssolvent wurde hier ein Lösungsmittelgemisch aus Dichloromethan/Aceton 1:1 verwendet.
Schmelzpunkt: 238°C

Elementaranalyse Berechnet (%) : C 54,01 H 4,53 N 1,96
Gefunden (%) : C 54,03 H 4,61 N 1,79

Spektroskopische Daten $^1$H-NMR (400 MHz, $CDCl_3$, δ): 5,49 (dd, H-1'), 5,32 (d, J = 7,0 Hz, NHCO), 5,20 (dd, H-4'), 5,12 (H-7), 4,33 (m, H-5' und H-3'), 4,27 (s, H-10), 3,68 (s, $OCH_3$).

Beispiel 5:

7-O-(3'-N-Trifluoroacetyl-α-L-daunosaminyl)-ε-isorhodomycinon (Verbindung 5)

200 mg (0,24 mmol) Verbindung 3 wurden in 20 ml Methanol gelöst und bei Raumtemperatur mit 0,1 ml wässriger 0,1n NaOH-Lösung versetzt. Nach 10 Minuten wurde der Reaktionsansatz mit 0,1 ml wässriger 0,1n HCl-Lösung neutralisiert. Das nach Eindampfen des Lösungsmittels erhaltene Produkt wurde anschließend über eine kurze Kieselgelsäule gerei nigt. Ausbeute der Titelverbindung 5: 149 mg (93%)

Elementaranalyse Berechnet (%) : C 53,81 H 4,51 N 2,09 F 8,51
Gefunden (%) : C 54,01 H 4,52 N 2,03 F 8,38

Beispiel 6:

7-O-α-L-Daunosaminyl-ε-isorhodomycinon (Verbindung 6)

Die Verbindung 5 wurde entsprechend der in Beispiel 5 beschriebenen Vorschrift, jedoch unter Anwendung von 0,5n NaOH zur Verbindung 6 entblockiert.
Schmelzpunkt: 153-155°C

Elementaranalyse Berechnet (%) : C 58,64 H 5,45 N 2,44
Gefunden (%) : C 58,37 H 5,51 N 2.32

Spektroskopische Daten $^1$H-NMR (270 MHz, $CD_3OD$,δ): 7,18 (H-2, H-3), 5,47 (H-1'), 5,11 (H-7), 4,2 (H-10), 3,73 ($COOCH_3$).
IR: (KBr, Γ, $cm^{-1}$) : 1720, 1590

Beispiel 7:

7-O-(3'-N-Acetyl-4'-O-acetyl-α-L-daunosaminyl)-ε-isorhodomycinon (Verbindung 7)

Verbindung 6 wurde nach dem in der Kohlenhydratchemie üblichen Acetylierungsverfahren mit Acetanhydrid/Pyridin/Dichlormethan in das 3'-N, 4'-O-Diacetyl-Derivat überführt.

Spektroskopische Daten $^1$H-NMR (400 MHz, $CDCl_3$, $\delta$): 5,49 (H-1'), 5,32 (J(NH, 3') = 7 Hz, NHCO), 5,20 (H-4'), 5,12 (H-7), 4,33 (m, H-5', H-3'), 4,27 (s, 10-H), 3,68 ($OCH_3$), 2,19 (OAc), 2,17 (NAc).

Beispiel 8:

7-O-(4'-O-Acetyl-3'-N-methyl-α-L-daunosaminyl)-ε-isorhodomycinon (Verbindung 8).

Eine Lösung von 7-O-(4'-O-Acetyl-α-L-rhodosaminyl)-ε-isorhodomycinon (Verbindung 1) (50 mg = 0,078 mmol) in einer Mischung aus Chloroform (100 ml) und Methanol (5 ml) wurde bei 0°C in einer Petrischale vom Durchmesser 19 cm auf einer reflektierenden Unterlage aus ca. 25 cm Abstand mit einem 500 Watt-Strahler (Schiansky) 1 Stunde lang belichtet. Dann wurde das Lösungsmittel am Rotationsverdampfer abgezogen, der Rückstand in möglichst wenig Methanol gelöst, mit Wasser versetzt, mit 1 %iger Salzsäure auf pH 4,4 eingestellt und das Methanol am Rotationsverdampfer entfernt. Die verbliebene wässrige Lösung wurde dreimal mit Chloroform extrahiert. Die vereinigten Chloroform-Phasen wurden dreimal mit Wasser vom pH 4,5 und danach einmal mit Wasser vom pH 9,5 rückextrahiert, über Natriumsulfat getrocknet, filtriert und das Lösungsmittel am Rotationsverdampfer abgezogen. Der Rückstand wurde durch wiederholtes Lösen in Dichlormethan und Fällen durch Zusatz von Hexan gereinigt und dünnschichtchromatographisch detektiert.
Ausbeute der Titelverbindung 8: 26 mg (0,041 mmol) = 53%.

Spektroskopische Daten: $^1$H-NMR (400 MHz, $CDCl_3$): 1,13 (t, 3H, J = 7,3 Hz, $CH_3$-14), 1,20 (d, 3H, $J_{5,6'}$ = 6,5 Hz, $CH_3$-6'), 2,18 (s, 3H, acetyl-$CH_3$), 2,33 (s, 3H, N-$CH_3$), 2,83 (bd, 1H, $J_{3',2'a}$ = 11,7 Hz, H-3'), 3,71 (s, 3H, $COOCH_3$) 4,21 (q, 1H, $J_{5',6'}$ = 6,5 Hz, H-5'), 4,29 (s, 1H, H-10), 4,38 (s, 1H, OH-9) 5,18 (s, 1H, H-4'), 5,25 (d, 1H, $J_{7,8}$ = 2,2 Hz, H-7), 5,52 (d, 1H, $J_{1',2'}$ = 3,4 Hz, H-1'), 7,29 (s, 2H, H-2 und H-3).

Beispiel 9:

7-O-(3'-N-Methyl-α-L-daunosaminyl)-ε-isorhodomycinon (Verbindung 9)

Eine Lösung von 7-O-α-L-Rhodosaminyl-ε-isorhodomycinon (Verbindung 2) (62 mg = 0,103 mmol) in einer Mischung aus Chloroform/Methanol (20/1) (310 ml) wurde in Analogie zu Beispiel 8 photolytisch demethyliert, wobei die Umsetzung dünnschichtchromatographisch verfolgt wurde. Nach Verbrauch der Ausgangsverbindung wurde das Lösungsmittel am Rotationsverdampfer abgezogen und das Reaktionsprodukt wiederholt säulenchromatographisch (10 g Kieselgel 60 für HPLC, 25-40 µm, Merck; Laufmittel: Dichlormethan/Methanol/Wasser (80/8/1) getrennt. Zur Entfernung von Kieselgel-Resten wurde das gereinigte Festprodukt mehrmals mit Chloroform extrahiert und aus den vereinigten Chloroformphasen zurückgewonnen.
Ausbeute der Titelverbindung 9: 27 mg (nicht optimiert)

Spektroskopische Daten: $^1$H-NMR (270 MHz, $CHCl_3$): 1,13 (t, 3H, J = 7,3 Hz, $CH_3$-14), 1,37 (d, 3H, $J_{5',6'}$ = 6,5 Hz, $CH_3$-6'), 2,39 (bs, 3H, N-$CH_3$), 2,81 (bd, 1H, $J_{2',3'}$ ca. 10 Hz, H-3'), 3,72 (s, 3H,$COOCH_3$), 4,10 (q, $J_{5'6'}$ = 6,5 Hz, H-5'), 4,28 (s, 1H, H-10), 4,43 (bs, 1H, OH-9), 5,25 (d, 1H, $J_{7,8}$ = 2,0 Hz, H-7), 5,49 (d, 1H, $J_{1',2'}$ = 2,5 Hz, H-1'), 7,30 (s, 2H, H-2 und H-3).

Der Molpeak des Massenspektrums (M + $H^{\oplus}$ = 588) steht im Einklang mit der berechneten Molmasse von 587,6 ($C_{29}H_{33}NO_{12}$).

Beispiel 10:

7-O-(3'-N-Acetyl-3'-N-methyl-α-L-daunosaminyl)-ε-isorhodomycinon (Verbindung 10)

Eine Lösung von 7-O-(4'-O-Acetyl-3'-N-methyl-α-L-daunosaminyl)-ε-isorhodomycinon (15 mg = 0,024 mmol) in trockenem Methanol wurde in Gegenwart katalytischer Mengen Natriummethylat zum 3'-N-Acetyl-Produkt isomerisiert und die Umsetzung dünnschichtchromatographisch verfolgt. Nach beendeter Reaktion wurde mit stark saurem Ionenaustauscher Dowex 50 WX8 neutralisiert, filtriert und das Lösungsmittel am Rotationsverdampfer abgezogen.

Spektroskopische Daten: $^1$H-NMR (400 MHz, $CDCl_3$): 1,13 (t, 3H, J = 7,2 Hz, $CH_3$-14), 1,28 (d, 3H, $J_{5',6'}$ = 6,4 Hz, $CH_3$-6'), 1,47 (m, 1H, H-13α), 1,84 (m, 1H, H-13 β), 2,04 (s, 3H, N-CO-$CH_3$), 2,1-2,4 (m, $CH_2$-8), 2,99 (s, 3H, N-$CH_3$), 3,71 (s, 3H, $COOCH_3$), 3,81 (bs, 1H, H-4'), 4,17 (1, 1H, H-5'), 4,30 (s, 1H, H-10), 5,24 (bs, 1H, H-7), 5,55 (bs, 1H, H-1'), 7,31 (s, 2H, H-2 und H-3).

Beispiel 11:

7-O-α-L-Rhodosaminyl-ε-isorhodomycinon (Verbindung 2)

30 mg (0.05 mmol) Verbindung 6 wurde in 10 ml Methanol gelöst. Nach der Zugabe von 0,1 ml einer 37 %igen wässrigen Formaldehyd-Lösung und 30 mg Natriumcyanoborhydrid wurde der Reaktionsansatz 3 d gerührt. Es wurde anschließend zur Trockne eingedampft und der resultierende Rückstand chromatographisch über Kieselgel gereinigt. Man erhielt 34 mg Verbindung 2. Die HPLC-Untersuchung ergab, daß diese Verbindung mit der in Beispiel 2 beschriebenen Verbindung 2 identisch war.

**Ansprüche**

1. Neue zytostatisch wirksame Anthracyclinderivate der allgemeinen Formel I

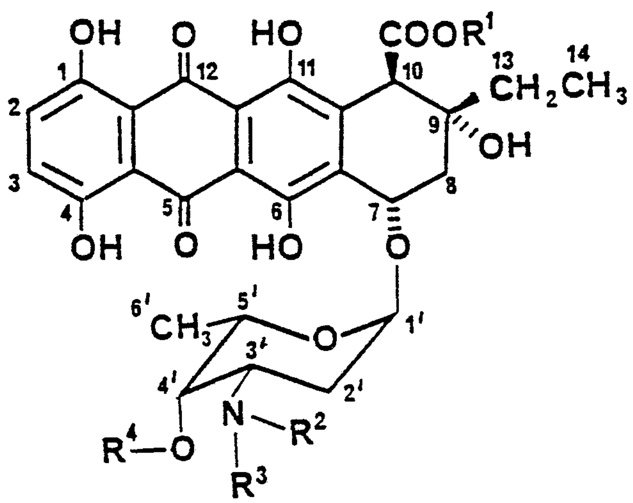

I

in der die Reste
$R^1$ eine $CH_3(CH_2)_n$ Gruppe mit n = 0 bis 3,
$R^2$ ein Wasserstoffatom oder eine Methylgruppe,
$R^3$ ein Wasserstoffatom, eine Methylgruppe oder eine Acylschutzgruppe
$R^4$ ein Wasserstoffatom oder eine Acylschutzgruppe bedeuten, und die gegebenenfalls als Salz einer anorganischen oder organischen Säure vorliegen.

2. Anthracyclinderivate nach Anspruch 1, **dadurch gekennzeichnet,** daß in der Formel I der Rest
$R^1$ eine Methylgruppe
$R^2$ ein Wasserstoffatom oder eine Methylgruppe
$R^3$ ein Wasserstoffatom oder eine Methylgruppe, eine Trifluoracetyl-oder Acetylgruppe und
$R^4$ ein Wasserstoffatom, eine Acetyl-, Benzoyl-oder para-Nitrobenzoylgruppe bedeuten.

3. Die Verbindung 7-O-(4'-O-Acetyl-α-L-rhodosaminyl)-ε-isorhodomycinon.

4. Die Verbindung 7-O-(α-L-Rhodosaminyl)-ε-isorhodomycinon.

5. Die Verbindung 7-O-(3'-N-Trifluoroacetyl-α-L-daunosaminyl)-ε-isorhodomycinon.

6. Die Verbindung 7-O-(4'-O-Acetyl-3'-N-methyl-α-L-daunosaminyl)-ε-isorhodomycinon.

7. Die Verbindung 7-O-(3'-N-Methyl-α-L-daunosaminyl)-ε-isorhodomycinon.

8. Verfahren zur Herstellung von Anthracyclinderivaten nach Anspruch 1, **dadurch gekennzeichnet,** daß

a) ein Anthracyclinon der allgemeinen Formel II,

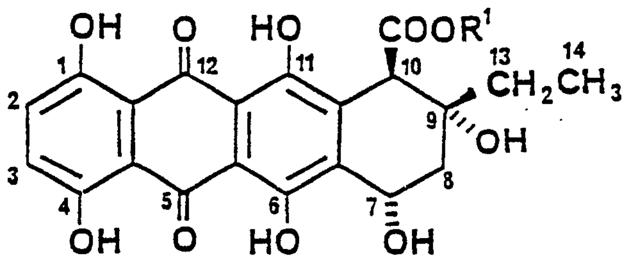

II

in der $R^1$ eine $CH_3(CH_2)_n$-Gruppe mit n gleich O bis 3 ist, mit einem funktionalisierten Daunosamin-Derivat der allgemeinen Formel III,

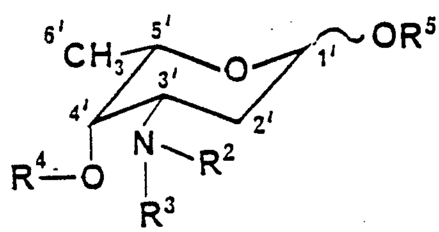

III

worin
$R^2$ ein Wasserstoffatom oder eine Methylgruppe,
$R^3$ eine Methylgruppe oder eine Acylschutzgruppe,
$R^4$ eine Acylschutzgruppe,
$R^5$ eine Acylschutzgruppe
bedeuten, in Gegenwart eines organischen Lösungsmittels, eines Katalysators, gegebenenfalls eines Säurefängers und eines Trockenmittels bei einer Reaktionstemperatur von -70°C bis +30°C zu einer Verbindung der allgemeinen Formel I umgesetzt wird, gegebenenfalls

b) bei einem Reaktionsprodukt der Stufe a) in an sich bekannter Weise die Schutzgruppen am Kohlenhydratsegment mittels einer anorganischen oder organischen Base in einem Lösungsmittel partiell oder vollständig entblockiert und gegebenenfalls wieder selektiv an der 4'-Hydroxy-und/oder 3'-Amino-Gruppe oder 3'-Methylamino-Gruppe eine neue Acylschutzgruppe eingeführt wird, und gegebenenfalls

c) bei einem in Stufe a) oder Stufe b) erhaltenen 3'-N,N-Dimethylamino-Reaktionsprodukt in an sich bekannter Weise auf photolytischem Wege in Gegenwart eines Lösungsmittels bei einer Reaktionstemperatur von +10°C bis +100°C eine am Stickstoff gebundene Methylgruppe abgespalten und gegebenenfalls das gebildete 3'-N-Methylamino-Derivat zu einem N-Acyl-Derivat oder einem N-Acyl-O-acyl-Derivat umgesetzt wird und gegebenenfalls

d) bei einem in Stufe b) erhaltenen Daunosamin-Reaktionsprodukt in an sich bekannter Weise eine N-Methylierung zum 3-N-Methyldaunosamin-Reaktionsprodukt oder eine N,N-Dimethylierung zum Rhodosamin-Reaktionsprodukt durchgeführt wird und gegebenenfalls

e) ein Produkt der Stufe a), b), c) oder d) in an sich bekannter Weise in das Salz einer anorganischen Säure oder organischen Säure überführt wird.

9. Verfahren nach Anspruch 8 , **dadurch gekennzeichnet,** daß ε-Isorhodomycinon mit 1,4-Di-O-Acetyl-α-L-rhodosamin in Gegenwart eines organischen Lösungs mittels, eines Katalysators, gegebenenfalls eines Säurefängers und eines Trockenmittels umgesetzt, gegebenenfalls der photolytischen Abspaltung einer Methylgruppe, der Desacylierung und gegebenenfalls der erneuten Acylierung und Salzbildung unterworfen wird.

10. Verfahren nach Anspruch 8 , **dadurch gekennzeichnet,** daß ε-Isorhodomycinon mit 1,4-Di-O-Acetyl-3-N-acetyl-α-L-daunosamin in Gegenwart eines organischen Lösungsmittels, eines Katalysators, gegebenenfalls eines Säurefängers und eines Trockenmittels umgesetzt, gegebenenfalls der Desacylierung und der N-Methylierung sowie gegebenenfalls der erneuten Acylierung und/oder Salzbildung unterworfen wird.

11. Verwendung eines Anthracyclin-Derivats nach Anspruch 1 in einem Arzneimittel.

Patentansprüche für die folgenden Vertragstaaten: GR, ES

1. Verfahren zur Herstellung von von zytostatisch wirksame Anthracyclinderivaten der allgemeinen Formel I

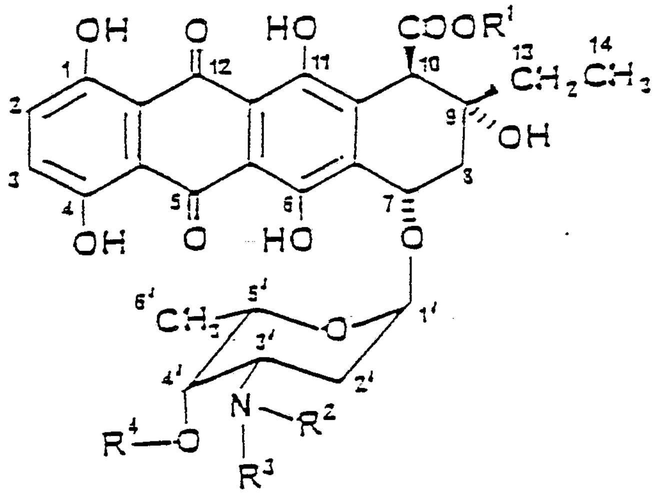

I

in der die Reste
$R^1$ eine $CH_3(CH_2)_n$ Gruppe mit n = 0 bis 3,
$R^2$ ein Wasserstoffatom oder eine Methylgruppe,
$R^3$ ein Wasserstoffatom, eine Methylgruppe oder eine Acylschutzgruppe
$R^4$ ein Wasserstoffatom oder eine Acylschutzgruppe bedeuten, und die gegebenenfalls als Salz einer anorganischen oder organischen Säure vorliegen **dadurch gekennzeichnet,** daß

a) ein Anthracyclinon der allgemeinen Formel II,

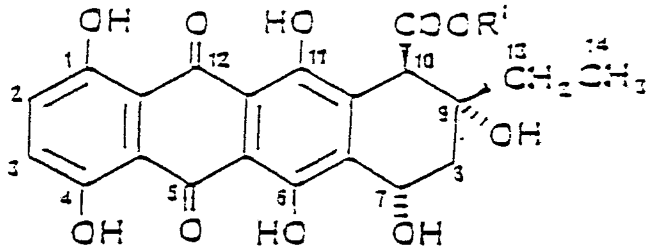

II

in der $R^1$ die vorstehend unter I angegebene Bedeutung hat, mit einem funktionalisierten Daunosamin-Derivat der allgemeinen Formel III,

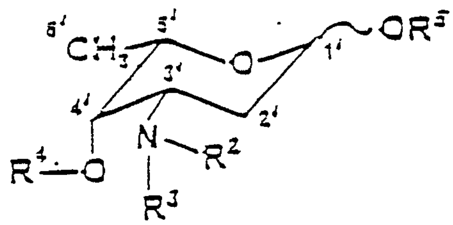

III

worin
$R^2$ ein Wasserstoffatom oder eine Methylgruppe,
$R^3$ eine Methylgruppe oder eine Acylschutzgruppe,
$R^4$ eine Acylschutzgruppe,
$R^5$ eine Acylschutzgruppe
bedeuten, in Gegenwart eines organischen Lösungsmittels, eines Katalysators, gegebenenfalls eines Säurefängers und eines Trockenmittels bei einer Reaktionstemperatur von -70°C bis +30°C zu einer Verbindung der allgemeinen Formel I mit $R^3$ und $R^4$ ungleich H umgesetzt wird, gegebenenfalls

b) bei einem Reaktionsprodukt der Stufe a) in an sich bekannter Weise die Schutzgruppen am Kohlenhydratsegment mittels einer anorganischen oder organischen Base in einem Lösungsmittel partiell oder vollständig entblockiert und gegebenenfalls wieder selektiv an der 4'-Hydroxy-und/oder 3'-Amino-Gruppe oder 3'-Methylamino-Gruppe eine neue Acylschutzgruppe eingeführt wird, und gegebenenfalls

c) bei einem in Stufe a) oder Stufe b) erhaltenen 3'-N,N-Dimethylamino-Reaktionsprodukt in an sich bekannter Weise auf photolytischem Wege in Gegenwart eines Lösungsmittels bei einer Reaktionstemperatur von +10°C bis +100°C eine am Stickstoff gebundene Methylgruppe abgespalten und gegebenenfalls das gebildete 3'-N-Methylamino-Derivat zu einem N-Acyl-Derivat oder einem N-Acyl-O-acyl-Derivat umgesetzt wird und gegebenenfalls

d) bei einem in Stufe b) erhaltenen Daunosamin-Reaktionsprodukt in an sich bekannter Weise eine N-Methylierung zum 3-N-Methyldaunosamin-Reaktionsprodukt oder eine N,N-Dimethylierung zum Rhodosamin-Reaktionsprodukt durchgeführt wird und gegebenenfalls

e) ein Produkt der Stufe a), b), c) oder d) in an sich bekannter Weise in das Salz einer anorganischen Säure oder organischen Säure überführt wird.

2. Verfahren nach Anspruch 1 ,
**dadurch gekennzeichnet,** daß ε-Isorhodomycinon mit 1,4-Di-O-Acetyl-α-L-rhodosamin in Gegenwart eines organischen Lösungs mittels, eines Katalysators, gegebenenfalls eines Säurefängers und eines Trockenmittels umgesetzt, gegebenenfalls der photolytischen Abspaltung einer Methylgruppe, der Desacylierung und gegebenenfalls der erneuten Acylierung und Salzbildung unterworfen wird.

3. Verfahren nach Anspruch 1,
**dadurch gekennzeichnet,** daß ε-Isorhodomycinon mit 1,4-Di-O-Acetyl-3-N-acetyl-α-L-daunosamin in Gegenwart eines organischen Lösungsmittels, eines Katalysators, gegebenenfalls eines Säurefängers und eines Trockenmittels umgesetzt, gegebenenfalls der Desacylierung und der N-Methylierung sowie gegebenenfalls der erneuten Acylierung und/oder Salzbildung unterworfen wird.

4. Verfahren zur Herstellung eines Arzneimittels, **dadurch gekennzeichnet**, daß ein nach Anspruch 1 erhaltenes Anthracyclin-Derivat verwendet wird.

Europäisches Patentamt

# EUROPÄISCHER RECHERCHENBERICHT

Nummer der Anmeldung

EP 87 11 7854

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 171 677 (SANRAKU)<br>* Seiten 4-8 *<br>--- | 1,11 | C 07 H 15/252<br>A 61 K 31/70 |
| A,D | CANADIAN JOURNAL OF CHEMISTRY, Band 58, Nr. 17, 1. September 1980, Seiten 1869-1874, National Research Council of Canada; J.M ESSERY et al.: "The synthesis of daunosaminyl epsilon-rhodomycinone, daunosaminyl 10-epi-epsilon-rhodomycinone, daunosaminyl epsilon-pyrromycinone, and 10-descarbomethoxy-epsilon-pyrromycin"<br>* Seite 1869, Tabelle; Seite 1872, Spalte 1, Zeile 24 - Spalte 2, Zeile 12 *<br>----- | 1,11 | |
| | | | **RECHERCHIERTE SACHGEBIETE (Int. Cl.4)** |
| | | | C 07 H 15/00<br>A 61 K 31/00 |

Der vorliegende Recherchenbericht wurde für alle Patentansprüche erstellt

| Recherchenort | Abschlußdatum der Recherche | Prüfer |
|---|---|---|
| DEN HAAG | 04-03-1988 | BRENNAN J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)